# FASCICULE DE BREVET EUROPEEN

(11) **EP 0 781 828 B1**
(45) Date de publication et mention de la délivrance du brevet: **12.07.2000**
(21) Numéro de dépôt: 96402768.4
(22) Date de dépôt: 17.12.1996
(51) Int. Cl.: C10G 9/16, C07C 4/04, C07C 11/22, B01J 19/00

(54) **Procédé de pyrolyse et de décokage en continu applicable notamment à la production d'acétylène**
Kontinuierliches Pyrolyse- und Entkohlungsverfahren, insbesonders zur Anwendung in der Herstellung von Acetylen
Continuous pyrolysis and decoking process, particularly applicable to the production of acetylene

(30) Priorité: 27.12.1995 FR 9515527
(43) Date de publication de la demande: 02.07.1997
(73) Titulaire: INSTITUT FRANCAIS DU PETROLE, 92852 Rueil-Malmaison Cedex (FR)
(72) Inventeur: Busson, Christian, 69260 Charbonniere (FR); Delhomme, Henri, 69110 Sainte Foy Les Lyon (FR)

(56) Documents cités:
- EP-A- 0 143 486
- EP-A- 0 539 270
- EP-A- 0 542 597
- EP-A- 0 591 856
- US-A- 1 470 359
- US-A- 3 641 190

## Description

L'invention concerne un procédé de pyrolyse d'une charge hydrocarbonée à au moins un atome de carbone et simultanément un procédé de décokage du coke déposé sur les parois du réacteur.

Elle s'applique notamment à la production en continu d'acétylène ou de composés acétyléniques comme le méthyl-acétylène.

L'art antérieur est notamment illustré par les brevet US-A-3,641,190, US-A-1,470,359, EP-A-0 591 856, EP-A-0 143 486, EP-A- 0 542 597 et EP-A-0 539 270.

Dans les procédés de transformation thermique à haute température d'hydrocarbures ayant au moins un atome de carbone, par exemple une pyrolyse entre 900 et 1 500 °C ou un vapocraquage vers 850 °C en fin de zone de chauffage, du coke se forme et se dépose à la surface des parois du réacteur. On réalise alors un décokage du réacteur qui se fait habituellement à l'air à des températures le plus souvent inférieures à 900 °C, en essayant dans le cas de fours métalliques d'éviter toute surchauffe ou points chauds préjudiciables à la bonne tenue des tubes métalliques du four. Ce décokage exothermique implique donc l'arrêt de toute l'unité et surtout la déconnexion du four des échangeurs thermiques en aval, ce qui réduit la productivité totale de l'unité. Par ailleurs, les règles de sécurité imposent le démontage des lignes d'introduction des hydrocarbures et leur remplacement par des lignes d'introduction de l'air, ce qui impose un arrêt très long de l'unité.

Lors du remontage de l'unité pour la phase de pyrolyse, les mêmes inconvénients demeurent auxquels s'ajoute la nécessité de purger la zone réactionnelle et les lignes avec un gaz inerte.

La pyrolyse d'hydrocarbures à au moins un atome de carbone permettant d'obtenir des composés hydrocarbonés oléfiniques ou acétyléniques a été décrite, notamment dans les demandes de brevet de la demanderesse FR 2715583 et FR 2732014 incorporées comme références.

On a notamment utilisé des réacteurs de pyrolyse en matière céramique dans lesquels des cloisons non étanches avantageusement en matière céramique déterminent des canaux où circulent la charge et les effluents réactionnels. Ces cloisons ont avantageusement une forme adaptée à créer des turbulences et comportent par exemple des alvéoles ou des cavités au niveau des moyens de chauffage. Ceux-ci sont en général des gaines contenant un chauffage électrique ou un brûleur à gaz.

Un objet de l'invention est de proposer un procédé permettant de pyrolyser une charge hydrocarbonée sans arrêt de l'unité pour permettre le décokage de cette unité.

Un autre objet de l'invention est de maintenir la température de l'installation sensiblement constante durant sa marche, pour éviter les contraintes thermiques qui ne manqueraient pas de se produire, notamment lors de l'utilisation d'un gaz contenant de l'oxygène pour l'étape de décokage qui met en oeuvre une réaction exothermique alors que l'étape de pyrolyse met en oeuvre une réaction endothermique.

Compte tenu de la présence des cloisons non étanches donc bon marché dans la zone réactionnelle, on a remarqué qu'il était possible de mettre en oeuvre en continu un procédé de pyrolyse d'une charge hydrocarbonée et un procédé de décokage de la zone réactionnelle qui ne soient pas pénalisants.

De manière détaillée l'invention concerne un procédé de pyrolyse et de décokage en continu pour produire un composé acétylénique dans une zone de réaction, en matière réfractaire, de forme allongée selon une direction (un axe) comprenant une zone de chauffage et une zone de refroidissement faisant suite à la zone de chauffage, la zone de chauffage comprenant au moins deux rangées sensiblement parallèles à l'axe séparées par une cloison, de préférence non étanche, en matériau réfractaire entre deux rangées successives, au moins l'une desdites rangées communiquant avec une alimentation en un mélange gazeux renfermant un hydrocarbure à au moins un atome de carbone et de la vapeur d'eau, au moins une autre desdites rangées communiquant avec une alimentation en un fluide non hydrocarboné comprenant de la vapeur d'eau, lesdites rangées comportant des moyens de chauffage entourés de gaines sensiblement parallèles entre elles et sensiblement perpendiculaires à l'axe du réacteur, du coke se déposant dans la zone de réaction, le procédé étant caractérisé en ce que l'on fait circuler le mélange gazeux dans au moins une rangée de la zone de chauffage de façon à pyrolyser ledit mélange pour former le composé acétylénique et à avoir une température de sortie de ladite zone d'au moins 1 000°C et on fait circuler ledit fluide comprenant de la vapeur d'eau dans au moins l'autre rangée de la zone de chauffage de façon à décoker au moins en partie la zone de réaction et à avoir une température de sortie de ladite zone d'au moins 1 000°C et l'on recueille des hydrocarbures et un effluent de décokage.

L'invention est particulièrement applicable dans le cas où la durée de pyrolyse avant décokage est supérieure et de préférence au moins deux fois supérieure à la durée de décokage.

La température dans la ou les rangées où s'effectue la pyrolyse est avantageusement maintenue sensiblement égale à la température dans la ou les rangées où s'effectue le décokage.

Le décokage des canaux à la vapeur d'eau à très haute température, supérieure à 1 000 °C de préférence de 1 000°C à 1 400 °C, sont des conditions suffisamment oxydantes à ces températures pour transformer le coke et pour former de l'oxyde de carbone et de l'hydrogène.

Ceci est particulièrement avantageux dans le cas des réacteurs en matière céramique comportant des cloisons non étanches. Un transfert de vapeur d'eau et d'hydrogène peut donc s'effectuer à travers la paroi de la rangée où s'effectue le décokage vers la rangée où s'effectue la pyrolyse.

Il a même été observé qu'un transfert d'hydrogène vers la rangée de pyrolyse ralentissait le dépôt de coke sur celle-ci.

Par ailleurs, un transfert de vapeur d'eau de la rangée où s'effectue le décokage vers la rangée où s'effectue la pyrolyse n'est pas pénalisant puisque la réaction de pyrolyse s'effectue en présence de vapeur d'eau. Dans l'autre sens, si des hydrocarbures passent de la rangée de pyrolyse vers la rangée où s'effectue le décokage, ils vont se trouver en présence de beaucoup d'eau et seront pyrolysés en produits recherchés.

Les moyens de chauffage peuvent être des résistances électriques contenues dans des gaines telles que décrites dans les brevets ci-devant ou ils peuvent être constitués de gaines contenant un brûleur à gaz tel que décrit dans la demande de brevet de la demanderesse (FR 2715583).

Chaque rangée peut comprendre au moins une nappe de moyens de chauffage entourés de gaines, sensiblement parallèle à l'axe de la zone de réaction, ces gaines étant sensiblement perpendiculaires audit axe.

Par ailleurs, il a été observé, qu'en présence d'éléments chauffants électriques contenus dans des gaines en matière céramique relativement poreuses et peu chères dont l'étanchéité n'est pas parfaite, un gaz de gaine contenant de l'hydrogène et/ou de la vapeur d'eau et/ou un gaz inerte pouvait être utilisé et de plus pouvait diffuser de l'intérieur vers l'extérieur des gaines sans perturber la réaction de pyrolyse et sans perturber la réaction de décokage.

Selon une caractéristique du procédé, le débit de vapeur d'eau introduite dans la rangée de décokage peut être 1,1 à 4 fois supérieur au débit d'eau introduit dans la rangée de pyrolyse. Le plus souvent, il est de 2 à 3 fois celui employé lors de la pyrolyse.

En d'autres termes, lors du décokage, on coupe l'alimentation en hydrocarbures dans la rangée destinée à être décokée et on augmente sensiblement le débit d'eau introduit de manière à ne pas provoquer de perturbation thermique trop forte dans le four de préchauffe des gaz en amont de la zone réactionnelle.

Selon une première variante, les hydrocarbures recueillis et l'effluent de décokage peuvent être mélangés avant d'être introduits dans la zone de refroidissement.

Selon une deuxième variante, les hydrocarbures recueillis et l'effluent de décokage sont refroidis séparément dans leurs rangées respectives, situées au niveau de la zone de refroidissement puis éventuellement mélangés.

La zone de refroidissement est habituellement une zone de trempe directe par un fluide de refroidissement, connu de l'Homme du métier.

Généralement, le débit d'eau dans la zone de pyrolyse est tel que le rapport pondéral eau/hydrocarbures est compris entre 0,55 et 20 et de préférence entre 0,65 et 10.

La pression est habituellement comprise entre 1 et 15 bars absolus (0,1 et 15 MPa) et de préférence comprise entre 1 et 3 bars absolus, le rapport pondéral étant plus particulièrement entre 0,8 et 5.

Les gaz préalablement chauffés sont introduits dans le réacteur à une température d'environ 400 à environ 700 °C, de préférence 500 à 650 °C.

Habituellement, la température finale en sortie de la zone de pyrolyse est comprise entre 1 000 °C et 1 400 °C.

Les caractéristiques des éléments chauffants, soit électriques, soit comportant des brûleurs à gaz, leur nombre, la distance les séparant et leur configuration sont décrits dans les brevets cités ci-devant.

Il en est de même pour les gaines les protégeant et les isolant des fluides qui circulent dans le réacteur.

Ces mêmes éléments chauffants et ces mêmes gaines avec les mêmes caractéristiques et les mêmes configurations se retrouvent aussi bien dans la zone de pyrolyse que dans la zone (ou rangée) où s'effectue le décokage à la vapeur d'eau.

L'invention concerne aussi un dispositif pour la mise en oeuvre du procédé.

Plus particulièrement, l'unité de pyrolyse et de décokage comporte un réacteur de pyrolyse de forme allongée selon une direction (un axe) comprenant aux moins deux rangées sensiblement parallèles à l'axe séparées par une cloison, de préférence non étanche, en matériau réfractaire entre deux rangées successives, chaque rangée comprenant une pluralité de moyens de chauffage disposés en au moins une nappe d'éléments chauffants entourés de gaines en matériau céramique sensiblement parallèles entre elles et sensiblement perpendiculaires à l'axe du réacteur, au moins l'une des rangées étant connectée à une ligne d'alimentation en une charge comportant au moins une vanne et à une ligne d'alimentation en vapeur d'eau comportant au moins une vanne, au moins une autre desdites rangées étant connectée à une ligne d'alimentation en un fluide non hydrocarboné contenant de la vapeur d'eau comportant au moins une vanne, ledit réacteur de pyrolyse comportant des moyens d'asservissement et de modulation de chauffage reliés aux moyens de chauffage, l'unité comprenant en outre des moyens de refroidissement des effluents produits dans chaque rangée.

L'unité présente l'avantage d'être sûre, fiable et facile à mettre en oeuvre. Elle utilise des matériaux réfractaires et plus particulièrement des matières céramiques connues de l'Homme du métier comme la cordiérite, la mullite, le nitrure de silicium ou le carbure de silicium.

Les charges hydrocarbonées utilisables sont, à titre d'exemples non limitatifs :
. les hydrocarbures aliphatiques saturés tels que le méthane, l'éthane, des mélanges d'alcanes (LPG), des coupes pétrolières telles que les naphtas, les gazoles atmosphériques et les gazoles sous vide, ces derniers pouvant présenter un point final de distillation de l'ordre de 570 °C .
. les hydrocarbures insaturés tels que l'éthylène, le propylène, le butadiène, des mélanges d'alcanes et d'alcènes tels que éthane + éthylène, les coupes C3, C4 et C5 de vapocraquage et de craquage catalytique.

Sur un plan industriel, les hydrocarbures préférés sont issus du vapocraquage et sont l'éthane pouvant contenir de l'éthylène en quantité très variable mais généralement assez faible, puis l'éthylène en mélange avec les autres hydrocarbures présents dans l'effluent du vapocraqueur.

L'invention sera mieux comprise par la description d'un mode de réalisation, donnée à titre purement illustratif mais nullement limitatif, qui en sera faite ci-après à l'aide de la figure annexée, qui représente une coupe longitudinale d'un réacteur suivant un plan parallèle à l'axe du réacteur.

Des lignes d'alimentation en hydrocarbures 11, 12, 13, 14, 15, 16 contrôlées par des vannes V1, V2, V3, V4, V5 et V6 introduisent dans un réacteur 40 de pyrolyse et de décokage des hydrocarbures provenant d'une ligne 10 en mélange avec de l'eau généralement sous forme vapeur apportée par une ligne 60. Cette ligne la distribue dans des lignes 17, 18, 19, 20, 21 et 22 contrôlées respectivement par des vannes V7, V8, V9, V10, V11 et V12.

Ces diverses vannes V1 à V12 sont adaptées à permettre la circulation d'un mélange hydrocarbures et vapeur d'eau dans un certain nombre de rangées du réacteur dites de pyrolyse et seulement de la vapeur d'eau dans d'autres rangées du réacteur dites de décokage pour y enlever le coke qui s'est déposé lors de la réaction de pyrolyse.

Des lignes 31, 32, 33, 34, 35, 36 transportant le mélange des hydrocarbures et de l'eau ou transportant l'eau seule, connectées respectivement aux lignes 11 et 22, 12 et 21, 13 et 20, 14 et 19, 15 et 18 et enfin, 16 et 17, sont préchauffées dans le four de préchauffe 30 à une température de 400 à 700 °C et sont connectées au réacteur de pyrolyse 40.

Par exemple, la vanne V1 fermant la ligne 11, il en résulte que la ligne 31 ne reçoit que de la vapeur d'eau amenée par la ligne 22 contrôlée par la vanne V12. En revanche, les lignes 32, 33, 34, 35 et 36 reçoivent le mélange hydrocarbures et eau, toutes les autres vannes mentionnées étant ouvertes.

Le réacteur 40 est divisé en rangées longitudinales (1, 2, 3, 4, 5 et 6) sensiblement parallèles à son axe. Ces rangées sont séparées les une des autres par des cloisons, 70, non étanches en matière céramique, de forme comportant des alvéoles adaptées à favoriser des turbulences à l'intérieur de la rangée et donc à favoriser la réaction. Ces rangées contiennent des gaines en matière céramique 7 formant une nappe sensiblement parallèle à l'axe du réacteur. Ces gaines sont sensiblement parallèles entre elles et sensiblement perpendiculaires à l'axe du réacteur. Elles contiennent, par exemple, une pluralité des résistances électriques 8 baignant dans un gaz de gaine, choisi dans le groupe formé par la vapeur d'eau, l'hydrogène, un gaz inerte et un mélange de deux ou plusieurs de ces gaz.

La ligne 31 de vapeur d'eau est connectée avec la rangée 1. Généralement, on augmente le débit de vapeur d'eau introduite dans la rangée où s'effectue le décokage, par exemple 2 à 3 fois celui utilisé dans les cinq autres rangées 2, 3, 4, 5 et 6 où s'effectue la pyrolyse. Ces rangées sont donc connectées respectivement aux lignes du mélange 32, 33, 34, 35 et 36.

La partie terminale des diverses rangées des réacteurs, destinée à la pyrolyse ou au décokage, reçoit les effluents de pyrolyse ou de décokage et chaque rangée est connectée à une ligne 47 de trempe directe, comprenant un injecteur à débit contrôlé, par exemple d'éthane si la charge est de l'éthane, ce qui permet de refroidir ces effluents. Une fois refroidis ves 800 °C par exemple, des lignes 41, 42, 43, 44, 45 et 46 connectées respectivement aux rangées 1, 2, 3, 4, 5 et 6 mélangent les divers effluents qui sont évacués par une ligne 50.

Selon un autre mode non illustré, les effluents peuvent être refroidis en circulant à travers des conduits étanches disposés dans la partie terminale des rangées par trempe indirecte puis mélangés comme décrit ci-dessus.

Selon un autre mode non illustré, les effluents de pyrolyse et de décokage issus des rangées 1, 2, 3, 4, 5 et 6 sont collectés par les lignes 41, 42, 43, 44, 45 et 46 puis mélangés et envoyés dans une zone de trempe directe ou indirecte et une fois refroidis évacués par la ligne 50.

Les éléments de chauffage 8 sont alimentés en énergie électrique de façon indépendante grâce à une paire d'électrodes non représentées sur la figure, des sondes pyrométriques à thermocouple non représentées sont logées dans les espaces où circule la charge et permettent de réguler automatiquement la température de chaque section de chauffage, par un dispositif classique de régulation et de modulation non représenté sur la figure, en fonction du profil de température choisi qui s'applique aussi bien à la réaction de pyrolyse qu'à celle de décokage des parois des gaines.

### Exemple

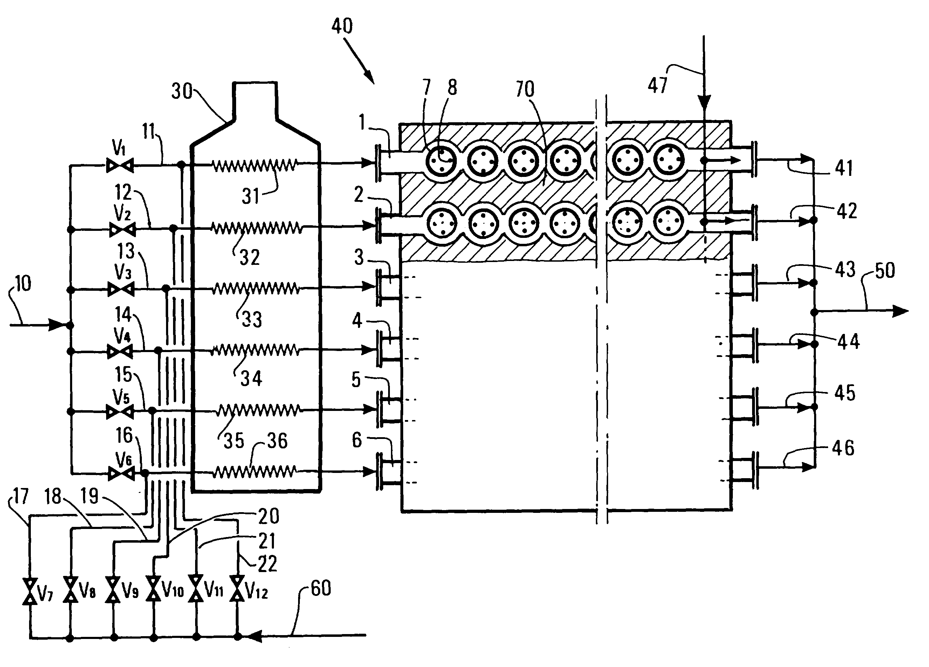

On utilise un réacteur décrit selon la figure 1 pour craquer un mélange d'éthane et de vapeur d'eau en vue de produire de l'acétylène.

Ce réacteur comporte 6 rangées de chauffage sensiblement parallèles à son axe et séparées par des cloisons en forme d'alvéoles et en matière céramique tel que le carbure de silicium par exemple. Chaque rangée comprend une nappe d'éléments chauffants électriques. Les gaines entourant les résistances électriques sont en carbure de silicum et contiennent un gaz de gaine qui est de l'azote.

Cinq rangées travaillent en pyrolyse (n° 1, 3, 4, 5 et 6) tandis qu'une seule rangée (n° 2) travaille en décokage.

Dans chaque rangée en pyrolyse, on introduit 258 kg/h d'éthane et 464 kg/h de vapeur d'eau.

Dans la rangée n° 2 opérant en décokage, la vanne V2 d'hydrocarbures étant fermée, on envoie 979 kg/h de vapeur d'eau par la vanne V11.

Le mélange (éthane-eau) à pyrolyser et la vapeur de décokage sont préchauffés à 630 °C et chauffés de manière sensiblement linéaire jusqu'à 1 200 °C dans leurs rangées respectives sous une pression absolue de 1,3 bar.

En sortie de réacteur de pyrolyse, l'effluent de pyrolyse est refroidi à 800 °C par contact direct avec 91 kg/h d'éthane à 16 °C tandis que l'effluent de décokage est refroidi à 800 °C par contact direct avec 85 kg/h d'éthane à 16 °C.

Après 72 heures de pyrolyse dans la rangée n° 1, on décide de procéder au décokage de celle-ci. Pour cela, on coupe le débit d'éthane par la vanne V1 et pour éviter une perturbation du régime thermique de la préchauffe et du four de pyrolyse, on augmente le débit de vapeur d'eau (vanne V12) jusqu'à obtenir 979 kg/h. Simultanément, on alimente à nouveau la rangée n° 2 avec 258 kg/h d'éthane et 464 kg/h de vapeur d'eau, en ouvrant la vanne V2 et la vanne V11.

On contrôle la fin du décokage par disparition du monoxyde de carbone, lequel est analysé en ligne par infra rouge par exemple en sortie de four de pyrolyse.

On constate que le décokage est complet au bout de 14 heures dans chaque rangée où il est réalisé et on repasse aussitôt en situation de réaction de pyrolyse pour la rangée qui a été décokée.

On a donc cinq rangées en pyrolyse et une rangée en décokage. On produit ainsi de manière constante et sans arrêt prolongé de l'unité 450 kg/h d'acétylène. Les effluents des six rangées sont mélangés et envoyés par la ligne 50 vers les traitements et les séparations des produits.

Bien évidemment, compte tenu de la durée de décokage adaptée à la charge choisie, on aurait pu disposer d'un réacteur comportant 10 rangées de pyrolyse et 2 rangées de décokage soit voisines soit séparées.

## Revendications

1. Procédé de pyrolyse et de décokage en continu pour produire un composé acétylénique dans une zone de réaction (40), en matière réfractaire, de forme allongée selon une direction (un axe) comprenant une zone de chauffage et une zone de refroidissement faisant suite à la zone de chauffage, la zone de chauffage comprenant au moins deux rangées (1, 2) sensiblement parallèles à l'axe séparées par une cloison (70) en matériau réfractaire entre deux rangées successives, au moins l'une desdites rangées (1) communiquant avec une alimentation (10, 60) en un mélange gazeux renfermant un hydrocarbure à au moins un atome de carbone et de la vapeur d'eau, au moins une autre (2) desdites rangées communiquant avec une alimentation (60) en un fluide non hydrocarboné comprenant de la vapeur d'eau, lesdites rangées comportant des moyens de chauffage (8) entourés de gaines (7) sensiblement parallèles entre elles et sensiblement perpendiculaires à l'axe du réacteur, du coke se déposant dans la zone de réaction, le procédé étant caractérisé en ce que l'on fait circuler le mélange gazeux dans au moins une rangée de la zone de chauffage de façon à pyrolyser ledit mélange pour former le composé acétylénique et à avoir une température de sortie de ladite zone d'au moins 1000 °C et on fait circuler ledit fluide non hydrocarboné comprenant de la vapeur d'eau dans au moins l'autre rangée de la zone de chauffage de façon à décoker au moins en partie la zone de réaction et à avoir une température de sortie de ladite zone d'au moins 1000 °C et l'on recueille des hydrocarbures et un effluent de décokage.

2. Procédé selon la revendication 1 dans lequel la température est maintenue sensiblement égale dans chacune des rangées.

3. Procédé selon la revendication 1 ou 2, dans lequel le débit de vapeur d'eau introduite dans la rangée de décokage est 1,1 à 4 fois supérieur au débit de vapeur d'eau introduite dans la rangée de pyrolyse.

4. Procédé selon l'une des revendications 1 à 3, dans lequel les hydrocarbures recueillis et l'effluent de décokage sont mélangés avant d'être introduits dans la zone de refroidissement.

5. Procédé selon l'une des revendications 1 à 3, dans lequel les hydrocarbures recueillis et l'effluent de décokage sont refroidis séparément dans leurs rangées respectives au niveau de la zone de refroidissement puis éventuellement mélangés.

6. Procédé selon l'une des revendications 1 à 5, dans lequel on réalise un refroidissement direct, des hydrocarbures recueillis et de l'effluent de décokage à l'aide d'un fluide de refroidissement.

7. Procédé selon l'une des revendications 1 à 6, dans lequel les gaines contiennent au moins un gaz choisi dans le groupe formé par l'hydrogène, la vapeur d'eau et un gaz inerte.

8. Procédé selon l'une des revendications 1 à 7, dans lequel on fait circuler le mélange gazeux dans au moins une rangée de la zone de chauffage de façon à pyrolyser ledit mélange et à avoir une température de sortie de ladite zone de chauffage d'environ 1 000 à 1 400 °C et on fait circuler ledit fluide comprenant de la vapeur d'eau dans au moins l'autre rangée de la zone de chauffage de façon à décoker au moins en partie la zone de réaction et à avoir une température de sortie de ladite zone de chauffage d'environ 1 000 à 1 400 °C

9. Procédé selon l'une des revendications 1 à 8, dans lequel chaque rangée comprend au moins une nappe de moyens de chauffage entourés de gaines sensiblement parallèles à l'axe de la zone de réaction.

10. Procédé selon l'une des revendications 1 à 9, appliqué à la production d'acétylène.

11. Procédé selon l'une des revendications 1 à 9, appliqué à la production de méthylacétylène.

12. Unité de pyrolyse et de décokage pour la mise en oeuvre de procédé selon l'une des revendications 1 à 11, comportant un réacteur (40) de pyrolyse de forme allongée selon une direction (un axe) comprenant au moins deux rangées (1, 2) sensiblement parallèles à l'axe séparées par une cloison (70) en matériau réfractaire entre deux rangées successives, chaque rangée comprenant une pluralité de moyens de chauffage (8) disposés en au moins une nappe d'éléments chauffants entourés de gaines (7) en matériau céramique sensiblement parallèles entre elles et sensiblement perpendiculaires à l'axe du réacteur, au moins l'une des rangées (1) étant connectée à une ligne d'alimentation (10) en une charge comportant au moins une vanne (V1) et à une ligne d'alimentation (60) en vapeur d'eau comportant au moins une vanne(22) , au moins une autre (2) desdites rangées étant connectée à une ligne d'alimentation (60) en à un fluide non hydrocarboné contenant de la vapeur d'eau comportant au moins une vanne (21), ledit réacteur de pyrolyse comportant des moyens d'asservissement et de modulation de chauffage reliés aux moyens de chauffage, l'unité comprenant en outre des moyens de refroidissement (47) des effluents produits dans chaque rangée.

## Patentansprüche

1. Verfahren zur kontinuierlichen Pyrolyse und Entkokung zur Erzeugung einer acetylenischen Verbindung in einer Reaktionszone (40) aus feuerfestem Material, von in einer Richtung (einer Achse) länglicher Gestalt, eine Heizzone und eine Kühlzone anschließend an diese Heizzone umfassend, wobei die Heizzone wenigstens zwei (1, 2) Reihenlagen, im wesentlichen parallel zur Achse und getrennt durch eine Trennungsanordnung (70) aus feuerfestem Material zwischen zwei aufeinanderfolgenden Reihenlagen, umfasst, wobei wenigstens eine dieser Reihenlagen (1) mit einer Zufuhr (10, 60) für ein gasförmiges Gemisch in Verbindung steht, das einen Kohlenwasserstoff mit wenigstens einem Kohlenstoffatom und Wasserdampf einschließt, und wenigstens eine andere (2) dieser Reihenlagen mit einer Zufuhr (60) für ein nicht kohlenwasserstoffhaltiges Wasserdampf umfassendes Fluid, in Verbindung steht, die Reihenlagen von Hüllen (7) umhüllte Heizmittel (8) umfassen, die im wesentlichen parallel zueinander und im wesentlichen senkrecht zur Achse des Reaktors sind, und Koks sich in der Reaktionszone absetzt, dadurch gekennzeichnet, dass man das gasförmige Gemisch in wenigstens einer Reihenlage der Heizzone zirkulieren lässt, um dieses Gemisch unter Bildung der acetylenischen Verbindung zu pyrolysieren und man über eine Austrittstemperatur aus dieser Zone von wenigstens 1000°C verfügt, und dass man dieses Wasserdampf enthaltende nicht kohlenwasserstoffhaltige Fluid in wenigstens einer anderen Reihenlage der Heizone zirkulieren lässt, derart, dass wenigstens zum Teil die Reaktionszone entkokt wird und man über eine Austrittstemperatur aus dieser Zone von wenigstens 1000°C verfügt und man Kohlenwasserstoffe und einen Entkokungsabstrom gewinnt.

2. Verfahren nach Anspruch 1, bei dem die Temperatur im wesentlichen gleich in sämtlichen Reihenlagen gehalten wird.

3. Verfahren nach Anspruch 1 oder 2, bei dem die in die Entkokungsreihenlage eingeführte Wasserdampfmenge um 1,1 bis 4-fach größer als die in die Pyrolysereihenlage eingeführte Wasserdampfmenge ist.

4. Verfahren nach einem der Ansprüche 1 bis 3, bei dem die gesammelten Kohlenwasserstoffe und der Entkokungsabstrom gemischt werden, bevor sie in die Kühlzone eingeführt werden.

5. Verfahren nach einem der Ansprüche 1 bis 3, bei dem die gesammelten Kohlenwasserstoffe und der Entkokungsabstrom getrennt in ihren jeweiligen Reihenlagen in Höhe der Kühlzone gekühlt, dann gegebenenfalls vermischt werden.

6. Verfahren nach einem der Ansprüche 1 bis 5, bei dem man eine direkte Kühlung der gesammelten Kohlenwasserstoffe und des Entkokungsabstroms mit Hilfe eines Kühlfluids realisiert.

7. Verfahren nach einem der Ansprüche 1 bis 6, bei dem die Hüllen wenigstens ein Gas enthalten, das aus der durch Wasserstoff, Wasserdampf und Inertgas gebildeten Gruppe gewählt ist.

8. Verfahren nach einem der Ansprüche 1 bis 7, bei dem man das gasförmige Gemisch in wenigstens einer Reihenlage der Heizzone derart zirkuliaren lässt, dass dieses Gemisch pyrolysiert wird und dass man über eine Austrittstemperatur aus der Heizzone von etwa 1000 bis 1400°C verfügt und man dieses Fluid, Wasserdampf umfassend, in wenigstens der anderen Reihenlage der Heizzone derart zirkulieren lässt, dass wenigstens zum Teil die Reaktionszone entkokt wird und man über eine Austrittstemperatur aus der Heizzone von etwa 1000 bis 1400°C verfügt.

9. Verfahren nach einem der Ansprüche 1 bis 8, bei dem jede Reihenlage wenigstens eine Bahn von Heizmitteln umfasst, die von Hüllen im wesentlichen parallel zur Achse der Reaktionszone umschlossen sind.

10. Verfahren nach einem der Ansprüche 1 bis 9, angewendet auf die Produktion von Acetylen.

11. Verfahren nach einem der Ansprüche 1 bis 9, angewendet auf die Produktion von Methylacetylen.

12. Pyrolyse- und Entkokungseinheit zur Durchführung des Verfahrens nach einem der Ansprüche 1 bis 11, einen Pyrolysereaktor (40) von in einer Richtung (einer Achse) länglicher Gestalt, der über wenigstens zwei Reihenlagen (1, 2), die im wesentlichen parallel zur Achse sind und durch eine Trennwand (70) aus feuerfestem Material zwischen zwei aufeinanderfolgenden Reihenlagen, getrennt sind, verfügt, wobei jede Reihenlage eine Vielzahl von Heizmitteln (8) umfasst, die in wenigstens einer Bahn von Heizelementen angeordnet sind, die von Hüllen (7) aus keramischem Material umschlossen sind, welche im wesentlichen parallel zueinander und im wesentlichen senkrecht zur Achse des Reaktors sind, wenigstens eine der Reihenlagen (1) mit einer Zuführleitung (10) für eine Charge, die über wenigstens ein Ventil (V1) verfügt und mit einer Wasserdampfzuführungsleitung (60) verbunden ist, die über wenigstens ein Ventil (22) verfügt, wobei wenigstens eine andere (2) dieser Reihenlagen mit einer Zuführleitung (60) für ein nicht-kohlenstoffhaltigen Fluid verbunden ist, welches Wasserdampf enthält und über wenigstens ein Ventil (21) verfügt und der Pyrolysereaktor Hilfssteuer- und Heizungsmodulationsmittel, verbunden mit den Heizmitteln, umfasst und die Einheit im übrigen Kühlmittel (47) für die in jeder Reihe erzeugten Abströme aufweist.

## Claims

1. A continuous pyrolysis and decoking process for producing an acetylenic compound, carried out in a reaction zone (40) which is of refractory material, which is elongate in one direction (one axis), and which comprises a heating zone and a cooling zone following the heating zone, the heating zone comprising at least two rows (1, 2) which are substantially parallel to the axis separated by a wall (70) of refractory material and located between two successive rows, at least one of said rows (1) communicating with a gaseous mixture supply (10, 60) containing a hydrocarbon containing at least one carbon atom and steam, at least one other (2) of said rows communicating with a supply (60) for a non hydrocarbon fluid comprising steam, said rows comprising heating means (8) surrounded by sleeves (7) which are substantially parallel to each other and substantially perpendicular to the reactor axis, wherein coke is deposited in the reaction zone, the process being characterized in that the gaseous mixture is circulated in at least one row of the heating zone to pyrolyse said mixture for producing an acetylenic compound and to produce a temperature at the outlet to said zone of at least 1000°C, and said fluid comprising steam is circulated in at least one other row of the heating zone to decoke the reaction zone at least in part and to produce a temperature at the outlet to said zone of at least 1000°C, and in which hydrocarbons and a decoking effluent are recovered.

2. A process according to claim 1, in which the temperature is maintained at the same temperature in each of the rows.

3. A process according to claim 1 or claim 2, in which the flow rate of the steam introduced into the decoking row is 1.1 to 4 times greater than the flow rate of steam introduced into the pyrolysis row.

4. A process according to any one of claims 1 to 3, in which the recovered hydrocarbons and the decoking effluent are mixed before being introduced into the cooling zone.

5. A process according to any one of claims 1 to 3, in which the recovered hydrocarbons and the decoking effluent are separately cooled in their respective rows in the cooling zone and may then be mixed.

6. A process according to any one of claims 1 to 5, in which direct cooling of the recovered hydrocarbons and the decoking effluent is carried out using a cooling fluid.

7. A process according to any one of claims 1 to 6, in which the sleeves contain at least one gas selected from the group formed by hydrogen, steam and an inert gas.

8. A process according to any one of claims 1 to 7, in which the gaseous mixture is circulated in at least one row of the heating zone to pyrolyse said mixture and produce a temperature at the outlet to said heating zone of about 1000°C to 1400°C and said fluid comprising steam is circulated in at least one other row of the heating zone to decoke the reaction zone at least partially, and to produce a temperature at the outlet to said heating zone of about 1000°C to 1400°C.

9. A process according to any one of claims 1 to 8, in which each row comprises at least one layer of heating means surrounded by sleeves which are substantially parallel to the reaction zone axis.

10. A process according to any one of claims 1 to 9, applied to the production of acetylene.

11. A process according to any one of claims 1 to 9, applied to the production of methyl acetylene.

12. A pyrolysis and decoking unit for carrying out the process of any one of claims 1 to 11, comprising a pyrolysis reactor (40) which is elongate in one direction (one axis) comprising at least two rows (1, 2) which are substantially parallel to the axis separated by a wall (70) of refractory material located between two successive rows, each row comprising a plurality of heating means (8) disposed in at least one layer of heating elements surrounded by sleeves (7) of ceramic material which are substantially parallel to each other and substantially perpendicular to the reactor axis, at least one of the rows (1) being connected to a feed supply line (10) comprising at least one valve (VI) and to a steam supply line (60) comprising at least one valve (22), at least one other (2) of said rows being connected to a supply line for a non hydrocarbon fluid containing steam and comprising at least one valve (21), said pyrolysis reactor comprising means for heat control and modulation connected to said heating means, the unit further comprising cooling means (47) for the effluents produced in each row.
